**Europäisches Patentamt**

**European Patent Office**

**Office européen des brevets**

(19)

(11) Veröffentlichungsnummer : **0 037 548**
**B1**

(12) # EUROPÄISCHE PATENTSCHRIFT

(45) Veröffentlichungstag der Patentschrift :
**21.03.84**

(21) Anmeldenummer : **81102403.3**

(22) Anmeldetag : **31.03.81**

(51) Int. Cl.³ : **C 07 D211/74**

(54) **Verfahren zur Herstellung von 2,2,6,6-Tetramethylpiperidon-4.**

(30) Priorität : **05.04.80 DE 3013403**

(43) Veröffentlichungstag der Anmeldung :
**14.10.81 Patentblatt 81/41**

(45) Bekanntmachung des Hinweises auf die Patenterteilung : **21.03.84 Patentblatt 84/12**

(84) Benannte Vertragsstaaten :
**BE CH DE FR GB IT LI NL**

(56) Entgegenhaltungen :
**DE-A- 2 429 937**
**DE-A- 2 916 471**

(73) Patentinhaber : **HOECHST AKTIENGESELLSCHAFT**
**Postfach 80 03 20**
**D-6230 Frankfurt am Main 80 (DE)**

(72) Erfinder : **Wiezer, Hartmut, Dr.**
**Hans-Fischer-Strasse 6**
**D-8906 Gersthofen (DE)**
Erfinder : **Nowy, Günther, Dr.**
**Hans-Fischer-Strasse 4**
**D-8906 Gersthofen (DE)**
Erfinder : **Häberlein, Harald, Dr.**
**Schlesierstrasse 2**
**D-8906 Gersthofen (DE)**

EP 0 037 548 B1

## Verfahren zur Herstellung von 2,2,6,6-Tetramethylpiperidon-4

2,2,6,6-Tetramethylpiperidon-4, welches auch als Triacetonamin bezeichnet wird und bevorzugt als Ausgangsmaterial für die Herstellung von sog. « Hindered Amine Light Stabilizers » dient, ist eine seit über 100 Jahren bekannte Verbindung. Sie entsteht durch Kondensation von Aceton mit Ammoniak unter dem katalytischen Einfluß von Lewis-Säuren oder Protonensäuren und deren Salzen mit Ammoniak oder Aminen (DE-B-16 95 753 (Deutsche Auslegeschrift) DE-A-24 29 935, 24 29 936, 24 29 937, 24 29 745, 24 29 746, 28 07 172, 29 10 761) (Deutsche Offenlegungsschriften).

Es wurde nun gefunden, daß man bei der Herstellung des 2,2,6,6-Tetramethylpiperidon-4 aus Aceton und Ammoniak überraschenderweise auch ganz andersartige Substanzen, nämlich teil- und perhalogenierte Kohlenwasserstoffe, mit bestem Erfolg als Katalysatoren einsetzen kann.

Die vorliegende Erfindung betrifft daher ein Verfahren zur Herstellung von 2,2,6,6-Tetramethylpiperidon-4 durch Umsetzen von Aceton mit Ammoniak bei erhöhten Temperaturen in Gegenwart von Katalysatoren und gegebenenfalls Cokatalysatoren und/oder Lösungsmitteln, welches dadurch gekennzeichnet ist, daß die Katalysatoren bei Raumtemperatur flüssige oder feste, teil- oder perhalogenierte, aliphatische oder cyclische Kohlenwasserstoffe mit 1 bis 28 bzw. 5 oder 6 C-Atomen, bei denen — sofern ihre Kohlenstoffatomzahl bei 2 bis 6 liegt — diese auch durch Doppelbindungen, jedoch keine polymerisierbaren, miteinander verknüpft sein können, sind.

Daß die erfindungsgemäß vorgeschlagenen Verbindungen in dem vorliegenden Falle als Katalysatoren brauchbar sein würden, war keineswegs vorhersehbar. Zum einen bestand aufgrund des umfangreichen Schrifttums über die Synthese des Triacetonamins, bei der, auch bei unterschiedlichsten Arbeitsbedingungen, stets sogenannte saure katalysatoren eingesetzt werden, das Vorurteil, daß nur mit diesen Katalysatoren 2,2,6,6-Tetramethylpiperidon-4 in technisch befriedigenden Ausbeuten zu erhalten sei, zum anderen war nicht damit zu rechnen, daß unter den Reaktionsbedingungen nachweislich inerte Substanzen wie Tetrachlorkohlenstoff oder Hexachlorethan katalytisch wirksam sein könnten [vgl. Ullmann Bd. 9 (1975), Seiten 416 und 475]. Des weiteren konnte nicht erwartet werden, daß das Verfahren auch noch mit einer erheblich besseren Selektivität als die bisher bekannten Verfahren das 2,2,6,6-Tetramethylpiperidon liefern würde : Während man nach dem Stand der Technik eine Selektivität von maximal 76 % erreicht (Deutsche Offenlegungsschrift 29 10 761), ist erfindungsgemäß eine mehr als 90 %ige Selektivität erzielbar. Es ergibt sich hieraus der technische Vorteil einer ökologisch geringeren Belastung. Von weiterem technischen Vorteil ist, daß bei Einsatz der bevorzugten Katalysatoren diese aus dem Reaktionsgemisch im allgemeinen abdestilliert und wiederverwendet werden können.

Geeignete Katalysatoren sind bei Raumtemperatur flüssige oder feste, teil- oder perhalogenierte, aliphatische oder cyclische Kohlenwasserstoffe, wobei unter Halogen bevorzugt Chlor sowie Brom und Jod zu verstehen sind, und auch unterschiedliche Halogenatome in einem Molekül enthalten sein können. In Frage kommen daher durch diese Halogene substituierte Aliphaten mit 1 bis ca. 28, vorzugsweise 1 bis 12 und insbesondere 1 oder 2 C-Atomen und auch solche mit 2 bis 6 C-Atomen, in denen Kohlenstoffatome durch Doppelbindungen, jedoch keine polymerisierbaren, miteinander verknüpft sind. Bevorzugt werden die perhalogenierten, insbesondere die perchlorierten Verbindungen. Beispiele sind 1,1,1,5-Tetrachlorpentan, Pentachlorethan, Tetrabromethan, Tetrachlorkohlenstoff, Tetrabromkohlenstoff, Chlorparaffine, Hexachlorethan, Trichlorethylen, Tetrachlorethan und Perchlorbutadien. Geeignete halogenhaltige cyclische Kohlenwasserstoffe besitzen 5 oder 6 C-Atome, wobei auch hier C-Atome durch nicht polymerisierbare Doppelbindungen miteinander verknüpft sein können. Genannt seien z. B. chlorierte Cyclohexane, Hexachlorbenzol und Hexachlorcyclopentadien. Als bevorzugt geeignet haben sich perchloriertes oder perbromiertes Mothan und die durch 4 bis 6 Chlor- oder Bromatome substituierten Ethane erwiesen, ganz besonders geeignet ist Tetrachlorkohlenstoff.

Die Katalysatoren werden in Mengen von 0,01 bis 5, vorzugsweise 0,1 bis 2 und insbesondere 0,1 bis 1 Mol-%, bezogen auf Aceton, eingesetzt. Man kann noch Cokatalysatoren in Mengen von 0,1 bis 0,5 %, bezogen auf Aceton, zugeben. Geeignet sind z. B. anorg. Isothiocyanate, Jodide, Bromide und Sulfide.

Das Molverhältnis Aceton : Ammoniak beträgt vorzugsweise 30 : 1 bis 2,5 : 1 und insbesondere 15 : 1 bis 4 : 1, die Reaktionstemperatur liegt bei 20 bis 120, vorzugsweise 50 bis 100 und insbesondere 70 bis 90 °C.

Das Ausgangsmaterial Aceton kann teilweise durch bei der Reaktion sich bildende, niedriger als das Verfahrensprodukt siedende Kondensationsprodukte des Acetons, wie Mesityloxid, Diacetonalkohol, Diacetonamin, Phoron, Acetonin (oder auch Triacetonamin, welches nach beendeter Reaktion zusammen mit den leichter siedenden Produkten in geringen Mengen abdestilliert wird), die auch geringe Wassermengen enthalten können, als Mischung oder als Einzelkomponente ersetzt werden, ohne daß dadurch Nachteile im Reaktionsverlauf entstehen. Nachdem somit das bei der Aufarbeitung anfallende Destillat von Leichtsiedern, welches neben Aceton aus den genannten Stoffen besteht, wieder einsetzbar ist, lassen sich nahezu quantitative Ausbeuten erzielen. Wenn sich nach wiederholtem Einsatz der Leichtsiederfraktion in dieser ein zu hoher Wassergehalt aufgebaut hat (mehr als ca. 5 Gew.-%), kann man diesen durch kurzzeitiges Verrühren des Destillats mit Alkalimetallhydroxid, gegebenenfalls in Form konzentrierter wäßriger Lösungen, wie z. B. Natronlauge, und Abtrennen der organischen Phase drastisch senken.

Das Verfahren kann in An- oder Abwesenheit organischer Lösungsmittel durchgeführt werden.

Genannt seien z. B. bei Raumtemperatur flüssige aliphatische und aromatische Kohlenwasserstoffe, Ether und Alkohole. Von besonderer Bedeutung sind mono- und polyfunktionelle Alkohole, bevorzugt jedoch Methanol oder Ethanol, weil sie nicht nur als Lösungsmittel dienen, sondern, bei Einsatz von 5 bis 30 Mol-%, bezogen auf Aceton, auch einen Lösungsvermittler und Überträger für das Ammoniakgas darstellen.

Das nach der erfindungsgemäßen Arbeitsweise in guten Ausbeuten erhältiche 2,2,6,6-Tetramethylpiperidon-4 dient, wie schon erwähnt, als Zwischenprodukt, u. a. bei der Herstellung von Piperidinstabilisatoren.

Die Erfindung soll durch nachstehende Beispiele weiter erläutert werden.

## Beispiel 1

In eine Mischung aus 2 500 g (43,1 Mol) Aceton, 150 g Methanol und 10 g frisch destilliertem Tetrachlorkohlenstoff (0,065 36 Mol = 0,151 6 Mol-% = 0,4 Gew.-%, bezogen auf Aceton) werden bei 10 bis 30 °C 116 g gasförmiger Ammoniak eingeleitet, was einem Molverhältnis Aceton : Ammoniak von 6,137 : 1 entspricht. Die Lösung füllt man in einen 5-Liter-Rührautoklaven aus Edelstahl, erhitzt 5 Stunden auf 80 °C und läßt zum Abkühlen stehen. Die Reaktionslösung enthielt, gaschromatographisch bestimmt, 3,9 % Acetonin (= 2,2,4,4,6-Pentamethyl-1,3,4,5-tetrahydropyrimidin), 18,8 % 2,2,6,6-Tetramethylpiperidon-4 und keine nicht verwertbaren hochsiedenden Nebenprodukte ($H_2O$ wird in der GC-Säule entzogen).

Die Rohlösung wird mit 150 g NaOH-Schuppen 30 min bei Raumtemperatur verrührt, die entstandene natronalkalische wäßrige Phase (350 g) abgetrennt und die organische Phase fraktioniert destilliert. Man erhält 450 g 2,2,6,6-Tetramethylpiperidon-4 (99,1 %ig nach GC). Zurück blieben 90 g eines harzigen Rückstandes.

Unter Berücksichtigung der Tatsache, daß die Leichsieder wiederverwendet werden, entspricht dies nach Destillation einer Selektivität von 96,5 %.

## Beispiele 2 bis 12

Mischungen aus jeweils 2 500 g Aceton und 150 g Methanol wurden mit Ammoniak unter Variieren der Ammoniakmenge, der Temperatur und der Katalysatoren wie in Beispiel 1 angegeben 5 Stunden lang zur Reaktion gebracht. Es wurde sodann wie beschrieben aufgearbeitet. Die Versuchsdaten sind in anliegender Tabelle zusammengestellt.

Die Aufarbeitung kann auch in der Weise vorgenommen werden, daß man aus der Rohlösung zunächst die niedriger als Triacetonamin siedenden Bestandteile im Wasserstrahlvakuum abdestilliert und den Rückstand sodann nach den Angaben der DE-OS 3 008 536 unter Zugabe eines unpolaren Lösungsmittels aus der Gruppe (cyclo) aliphatische Kohlenwasserstoffe, Tetrachlorethan und Tetrachlorkohlenstoff bei Temperaturen unter 25 °C kristallisiert. Diese Methode liefert ein besonders reines Triacetonamin.

(Siehe Tabelle, Seite 4 f.)

| Bsp. Nr. | $NH_3$ (g) $\hat{=}$ Mol-% [1] | Katalysator (g) $\hat{=}$ Mol-% [1] | Temperatur (°C) | Ausbeuten bezogen auf Aceton | |
|---|---|---|---|---|---|
| | | | | Triacetonamin g $\hat{=}$ % | Hochsieder g $\hat{=}$ % |
| 2 | 114 g / 6,4 | $CCl_4$ 10 g / 0,15 | 70 | 395 g $\hat{=}$ 17,7 % | 73 g $\hat{=}$ 3,3 % |
| 3 | 155 g / 4,73 | $CCl_4$ 50 g / 0,75 | 70 | 317 g $\hat{=}$ 14,2 % | 221 g $\hat{=}$ 9,9 % |
| 4 | 134 g / 5,47 | $CCl_4$ 50 g / 0,75 | 80 | 594 g $\hat{=}$ 26,7 % | 293 g $\hat{=}$ 13,2 % |
| 5 | 97 g / 7,55 | $(CCl_3)_2$ 10 g / 0,09 | 80 | 498 g $\hat{=}$ 22,4 % | 125 g $\hat{=}$ 5,6 % |
| 6 | 103 g / 7,11 | $(CHCl_2)_2$ 20 g / 0,28 | 80 | 524 g $\hat{=}$ 23,5 % | 115 g $\hat{=}$ 5,2 % |
| 7 | 100 g / 7,3 | $(CHBr_2)_2$ 20 g / 0,12 | ·80 | 501 g $\hat{=}$ 25,5 % | 153 g $\hat{=}$ 6,9 % |
| 8 | 100 g / 7,3 | $CCl_3 \cdot CHCl_2$ 20 g/ 0,23 | 80 | 460 g $\hat{=}$ 20,7 % | 130 g $\hat{=}$ 5,8 % |
| 9 | 90 g / 8,1 | $CCl_3(CH_2)_3CH_2Cl$ 20 g/0,22 | 80 | 235 g $\hat{=}$ 10,6 % | 45 g $\hat{=}$ 2,0 % |
| 10 | 100 g / 7,3 | $CCl_2=Cl-CCl=CCl_2$ 20 g/0,17 | 80 | 250 g $\hat{=}$ 11,2 % | 25 g $\hat{=}$ 1,1 % |
| 11 | 105 g / 6,79 | Hexachlor-Cyclopentadien 20 g / 0,17 | 80 | 365 g $\hat{=}$ 16,4 % | 300 g $\hat{=}$ 13,5 % |
| 12 | 100 g / 7,33 | Chloriertes Paraffin 70 % Chlor 10 g/0,017 | 80 | 474 g $\hat{=}$ 21,3 % | 120 g $\hat{=}$ 5,4 % |

1) Bezogen auf eingesetztes Aceton

0 037 548

Beispiel 13

2 500 g von bei den Beispielen 2 bis 4 abdestillierten Leichtsiedern (die den in diesen Ansätzen als Katalysator verwendeten Tetrachlorkohlenstoff enthielten), wurden mit 100 g NH$_3$ wie in Beispiel 1 angegeben bei 80 °C umgesetzt.

Ausbeute : 475 g Triacetonamin und 188 g Hochsieder.

Beispiel 14

500 g Toluol und 50 g Tetrachlorkohlenstoff wurden bei 100 °C 5 Stunden unter einem Ammoniakdruck von 10 bar gehalten. Nach dem Abdestillieren des gesamten Ansatzes verblieb keinerlei Rückstand, wodurch gezeigt wird, daß bei dem erfindungsgemäßen Verfahren der Tetrachlorkohlenstoff als solcher katalytisch wirkt und nicht etwa ein Spalt- oder Umsetzungsprodukt desselben, da ein solches unter den Verfahrensbedingungen augenscheinlich nicht entsteht.

**Ansprüche**

1. Verfahren zur Herstellung von 2,2,6,6-Tetramethylpiperidon-4 durch Umsetzen von Aceton mit Ammoniak bei erhöhten Temperaturen in Gegenwart von Katalysatoren und gegebenenfalls Cokatalysatoren und/oder Lösungsmitteln, dadurch gekennzeichnet, daß die Katalysatoren bei Raumtemperatur flüssige oder feste, teil- oder perhalogenierte, aliphatische oder cyclische Kohlenwasserstoffe mit 1 bis 28 bzw. 5 oder 6 C-Atomen, bei denen — sofern ihre Kohlenstoffatomzahl bei 2 bis 6 liegt — diese auch durch Doppelbindungen, jedoch keine polymerisierbaren, miteinander verknüpft sein können, sind.

2. Verfahren nach Anspruch 1, dadurch gekennzeichnet, daß die Katalysatormenge 0,01 bis 5 Mol-%, bezogen auf Aceton, beträgt, das Molverhältnis Aceton : Ammoniak bei 30 : 1 bis 2,5 : 1 liegt und man bei 20 bis 120 °C arbeitet.

3. Verfahren nach den Ansprüchen 1 und 2, dadurch gekennzeichnet, daß der Katalysator ein perchloriertes oder perbromiertes Methan oder ein durch 4 bis 6 Chlor- oder Bromatome substituiertes Ethan ist.

4. Verfahren nach den Ansprüchen 1 bis 3, dadurch gekennzeichnet, daß das Aceton teilweise durch die bei der Aufarbeitung vorheriger Ansätze anfallenden Leichtsieder als Mischung oder als Einzelkomponenten ersetzt wird.

**Claims**

1. A process for the preparation of 2,2,6,6-tetramethylpiperidone-4 by reaction of acetone with ammonia at elevated temperatures in the presence of catalysts and optionally cocatalysts and/or solvents, wherein the catalysts are partially halogenated or perhalogenated, aliphatic or cyclic hydrocarbons liquid or solid at room temperature and having from 1 to 28 or 5 or 6 carbon atoms, respectively ; these carbon atoms, in the case where their number is from 2 to 6, may also be linked with one another by nonpolymerizable double bonds.

2. The process as claimed in Claim 1, wherein the catalyst amount is from 0.01 to 5 mol-%, relative to acetone, the molar ratio of acetone to ammonia is from 30 : 1 to 2.5 : 1 and the operations are carried out at 20 to 120 °C.

3. The process as claimed in Claims 1 and 2, wherein the catalyst is a perchlorinated or perbrominated methane or an ethane substituted by 4 to 6 chlorine or bromine atoms.

4. The process as claimed in Claims 1 to 3, wherein the acetone is partially replaced by the low-boiling substances obtained in the work-up of previous batches, either in the form of a mixture or as individual components.

**Revendications**

1. Procédé de préparation de la tétraméthyl-2,2,6,6 pipéridone-4 par réaction de l'acétone avec l'ammoniac, à des températures élevées, en présence de catalyseurs et éventuellement de co-catalyseurs et/ou de solvants, procédé caractérisé en ce que les catalyseurs sont des hydrocarbures aliphatiques ou cycliques, respectivement en C$_1$-C$_{28}$ ou en C$_5$ ou C$_6$, partiellement halogénés ou perhalogénés, liquides ou solides à la température ambiante, dans lesquels — lorsqu'ils contiennent de 2 à 6 atomes de carbone — ceux-ci peuvent également être reliés entre eux par des doubles liaisons à condition que celles-ci ne soient pas polymérisables.

2. Procédé selon la revendication 1, caractérisé en ce que la quantité de catalyseur est comprise entre 0,01 et 5 % en moles par rapport à l'acétone, en ce que le rapport molaire de l'acétone à l'ammoniac est compris entre 30 : 1 et 2,5 : 1 et en ce qu'on opère à une température comprise entre 20 et 120 °C.

3. Procédé selon l'une des revendications 1 et 2, caractérisé en ce que le catalyseur est un méthane perchloré ou perbromé ou un éthane portant de 4 à 6 atomes de chlore ou de brome.

4. Procédé selon l'une quelconque des revendications 1 à 3, caractérisé en ce que l'acétone est remplacée partiellement par les corps à bas points d'ébullition que l'on obtient lors du traitement complémentaire de mélanges provenant d'opérations antérieures, ces corps à bas points d'ébullition étant mis en jeu isolément ou sous la forme de mélanges.